# EUROPÄISCHE PATENTSCHRIFT

(11) **EP 0 727 483 B1**
(45) Veröffentlichungstag und Bekanntmachung des Hinweises auf die Patenterteilung: **29.10.2008**
(21) Anmeldenummer: 96100770.5
(22) Anmeldetag: 19.01.1996
(51) Int. Cl.: C12N 7/00, C12N 15/49, C07K 14/16, G01N 33/569, C12Q 1/70, A61K 39/21

(54) **Retrovirus aus der HIV-Gruppe und dessen Verwendung**
Retrovirus of the HIV-group and its application
Rétrovirus du groupe HIV et son application

(30) Priorität: 16.02.1995 DE 19505262
(43) Veröffentlichungstag der Anmeldung: 21.08.1996
(73) Patentinhaber: Siemens Healthcare Diagnostics Products GmbH, 35041 Marburg (DE)
(72) Erfinder: Hauser, Hans-Peter, Dr., D-35041 Marburg (DE); Knapp, Stefan, Dr., D-35041 Marburg (DE); Brust, Stefan, Dr., D-35041 Marburg (DE); Gürtler, Lutz G., Prof. Dr., D-81249 München (DE); Eberle, Josef, Dr., D-85356 Freising (DE); Kaptue, Lazare, Prof., Yaoundé (CM); Zekeng, Léopold A., Dr., Yaoundé (CM)
(74) Vertreter: Keller, Günter

(56) Entgegenhaltungen:
- EP-A- 0 591 914
- WO-A-93/18054
- WO-A-96/12809
- WO-A-96/27013
- JOURNAL OF VIROLOGY, Bd. 68, Nr. 3, März 1994, Seiten 1586-1596, XP002034445 M.VANDEN HAESEVELDE ET AL.: "Genomic cloning and complete sequence analysis of a highly divergent African Human Immunodeficiency Virus isolate"
- VIROLOGY, Bd. 205, Nr. 1, 15.November 1994, ORLANDO US, Seiten 247-253, XP002005314 P.CHARNEAU ET AL.: "Isolation and envelope sequence of a highly divergent HIV-1 isolate: Definition of a new HIV-1 group"

### SEQUENZPROTOKOLL

(1) ALLGEMEINE INFORMATION:
   ANMELDER:
      (A) NAME: Behringwerke Aktiengesellschaft
      (B) STRASSE: Postfach 11 40
      (C) ORT: Marburg
      (E) LAND: Germany
      (F) POSTLEITZAHL: 35001
   ANMELDETITEL:
      Retrovirus aus der HIV-Gruppe und dessen Verwendung (MVP-2901/94)
   ANZAHL DER SEQUENZEN: 12
   COMPUTER-LESBARE FORM:
      (A) DATENTRÄGER: Floppy Disk
      (B) COMPUTER: IBM PC compatible
      (C) BETRIEBSSYSTEM: PC-DOS/MS-DOS
      (D) SOFTWARE: PADAT Sequenzmodul Version 1.0
(2) INFORMATION ZU SEQ ID NO: 1:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 1: AGTGCAGCAG GTAGCACTAT G 21
(2) INFORMATION ZU SEQ ID NO: 2:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 2: GTTCCATTTT ACTGATGTGT A 21
(2) INFORMATION ZU SEQ ID NO: 3:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 3: AGTGCAGCAG GTAGCACTAT G 21
(2) INFORMATION ZU SEQ ID NO: 4:
   (i) SEQUENZ CHARAKTERISTIKA:
      (A) LÄNGE: 22 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 4: TTTAGTTATG TCAAACCAAT TC 22
(2) INFORMATION ZU SEQ ID NO: 5:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 5: GTTCCATTTT ACTGATGTGT A 21
(2) INFORMATION ZU SEQ ID NO: 6:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 6: TCGGTACGAA CCCACTCAT 19
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 7: ACTATACCCC TCATTAATGA 20
(2) INFORMATION ZU SEQ ID NO: 8:
   (i) SEQUENZ CHARAKTERISTIKA:
      (A) LÄNGE: 23 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 8: AACTGTCATG GAGAATTCTT TTA 23
(2) INFORMATION ZU SEQ ID NO: 9:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 9: AGTAGTTACT TGTACACATG G 21
(2) INFORMATION ZU SEQ ID NO: 10:
   (i) SEQUENZ CHARAKTERISTIKA:
      (A) LÄNGE: 1070 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 10:
   TCAGGTAATA TCTTAGTGAC CCTAAATTCT ACTATAAACA TGACCTGCGT GAGGCCAGGA 60
   AATAATCCAG TACAGGAGAT AAGGATAGGT CCAATGGCTT GGTACAGTAT GGGACTTGAG 120
   AGAGGGTATA CAAATAAATC AAGAATAGCT TATTGTGCCT ATAATGTCAC AAAATGGAAA 180
   GAAACCTTGC AAGGGATAGC TGAAAGGTAT TTAGAACTTG TAAATTATTC AAGAAACATG 240
   ACCATAACAT TCAATAGCAG CATTGGTGGA GGAGATATAG AAGTAACCCG TTTGCATTTT 300
   AACTGTCATG GAGAATTCTT TTATTGTAAC ACAAGTCAAA TGTTTAATTA TACATTCAAA 360
   TGTAATAACT CCAAATGTAA TACTCATAAT GACAATAATA CTTATGAGAA CAGTACAAGA 420
   ATAATATATT GCCAGTTGAG ACAGGTAGTA AGGTCATGGA TGAGGGGAGG GTCAGGGCTC 480
   TATGCACCTC CTATCAGAGG TAATCTAACC TGCAATTCAA ACATAACTGG ATTGATTCTA 540
   CAAATGGATA CACCATATAA TAAAAGCTCC AACATCACAT TTAGACCAAT AGGAGGAGAT 600
   ATGAAGGATA TATGGAGAAC CCAAATGTAC AATTACAAAG TAGTAAGGGT AAAATCTTTT 660
   AGTGTAGCAC CTACTAAGAT TAGTAGACCA GTTATAGGCA CTAACCATCA AAGAGAAAAA 720
   AGGGCAGTAG GATTGGGAAT GCTATTCTTG GGGGTTCTAA GTGCAGCAGG TAGCACTATG 780
   GGCGCAGCGG GAGTAACGCT GTCGGTACGA ACCCACTCAT TAATGAGGGG TATAGTGCAA 840
   CAGCAGGACA ACCTGCTGAG AGCAATACAG GCCCAGCAAC ATCTGCTGAG GTTATCTGTA 900
   TGGGGTATTA GACAACTCCG AGCTCGCCTG CAAGCCTTAG AAACCCTTAT GCAGAATCAG 960
   CAACTCCTAA ACCTGTGGGG CTGTAAAGGA AAATTAATCT GCTACACATC AGTAAAATGG 1020
   AACGAAACAT GGGGAGGAAA TCTCTCAATT TGGGACAGCT TAACATGGCA
   1070
(2) INFORMATION ZU SEQ ID NO: 11:
   (i) SEQUENZ CHARAKTERISTIKA:
      (A) LÄNGE: 1070 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 11:
   AGTCCATTAT AGAATCACTG GGATTTAAGA TGATATTTGT ACTGGACGCA CTCCGGTCCT 60
   TTATTAGGTC ATGTCCTCTA TTCCTATCCA GGTTACCGAA CCATGTCATA CCCTGAACTC 120
   TCTCCCATAT GTTTATTTAG TTCTTATCGA ATAACACGGA TATTACAGTG TTTTACCTTT 180
   CTTTGGAACG TTCCCTATCG ACTTTCCATA AATCTTGAAC ATTTAATAAG TTCTTTGTAC 240
   TGGTATTGTA AGTTATCGTC GTAACCACCT CCTCTATATC TTCATTGGGC AAACGTAAAA 300
   TTGACAGTAC CTCTTAAGAA AATAACATTG TGTTCAGTTT ACAAATTAAT ATGTAAGTTT 360
   ACATTATTGA GGTTTACATT ATGAGTATTA CTGTTATTAT GAATACTCTT GTCATGTTCT 420
   TATTATATAA CGGTCAACTC TGTCCATCAT TCCAGTACCT ACTCCCCTCC CAGTCCCGAG 480
   ATACGTGGAG GATAGTCTCC ATTAGATTGG ACGTTAAGTT TGTATTGACC TAACTAAGAT 540
   GTTTACCTAT GTGGTATATT ATTTTCGAGG TTGTAGTGTA AATCTGGTTA TCCTCCTCTA 600
   TACTTCCTAT ATACCTCTTG GGTTTACATG TTAATGTTTC ATCATTCCCA TTTTAGAAAA 660
   TCACATCGTG GATGATTCTA ATCATCTGGT CAATATCCGT GATTGGTAGT TTCTCTTTTT 720
   TCCCGTCATC CTAACCCTTA CGATAAGAAC CCCCAAGATT CACGTCGTCC ATCGTGATAC 780
   CCGCGTCGCC CTCATTGCGA CAGCCATGCT TGGGTGAGTA ATTACTCCCC ATATCACGTT 840
   GTCGTCCTGT TGGACGACTC TCGTTATGTC CGGGTCGTTG TAGACGACTC CAATAGACAT 900
   ACCCCATAAT CTGTTGAGGC TCGAGCGGAC GTTCGGAATC TTTGGGAATA CGTCTTAGTC 960
   GTTGAGGATT TGGACACCCC GACATTTCCT TTTAATTAGA CGATGTGTAG TCATTTTACC 1020
   TTGCTTTGTA CCCCTCCTTT AGAGAGTTAA ACCCTGTCGA ATTGTACCGT 1070
(2) INFORMATION ZU SEQ ID NO: 12:
   (i) SEQUENZ CHARAKTERISTIKA:
      (A) LÄNGE: 356 Aminosäuren
      (B) ART: Aminosäure
      (C) STRANGFORM: nicht bekannt
      (D) TOPOLOGIE: nicht bekannt
   (ii) ART DES MOLEKÜLS: Protein
   (v) ART DES FRAGMENTS: inneres
   (xi) SEQUENZBESCHREIBUNG: SEQ ID NO: 12:
   Ser Gly Asn Ile Leu Val Thr Leu Asn Ser Thr Ile Asn Met
   Thr Cys
        1               5                   10
   15
   Val Arg Pro Gly Asn Asn Pro Val Gln Glu Ile Arg Ile Gly
   Pro Met
                     20                  25                  30
   Ala Trp Tyr Ser Met Gly Leu Glu Arg Gly Tyr Thr Asn Lys
   Ser Arg
                 35                  40                  45
   Ile Ala Tyr Cys Ala Tyr Asn Val Thr Lys Trp Lys Glu Thr
   Leu Gln
             50                  55                  60
   Gly Ile Ala Glu Arg Tyr Leu Glu Leu Val Asn Tyr Ser Arg
   Asn Met
        65                  70                  75
   80
   Thr Ile Thr Phe Asn Ser Ser Ile Gly Gly Gly Asp Ile Glu
   Val Thr
                         85                  90
   95
   Arg Leu His Phe
               100
   Asn Cys His Gly Glu Phe Phe Tyr Cys Asn Thr Ser Gln Met
   Phe Asn
                       105                 110
   115
   Tyr Thr Phe Lys Cys Asn Asn Ser Lys Cys Asn Thr His Asn
   Asp Asn
                   120                 125                 130
   Asn Thr Tyr Glu Asn Ser Thr Arg Ile Ile Tyr Cys Gln Leu Arg Gln
   135 140 145
   Val Val Arg Ser Trp Met Arg Gly Gly Ser Gly Leu Tyr Ala Pro Pro
   150 155 160
   Ile Arg Gly Asn Leu Thr Cys Asn Ser Asn Ile Thr Gly Leu Ile Leu
   165 170 175 180
   Gln Met Asp Thr Pro Tyr Asn Lys Ser Ser Asn Ile Thr Phe Arg Pro
   185 190 195
   Ile Gly Gly Asp Met Lys Asp Ile Trp Arg Thr Gln Met Tyr Asn Tyr
   200 205 210
   Lys Val Val Arg Val Lys Ser Phe Ser Val Ala Pro Thr Lys Ile Ser
   215 220 225
   Arg Pro Val Ile Gly Thr Asn His Gln Arg Glu Lys Arg Ala Val Gly
   230 235 240
   Leu Gly Met Leu Phe Leu Gly Val Leu Ser Ala Ala Gly Ser Thr Met
   245 250 255 260
   Gly Ala Ala Gly Val Thr Leu Ser Val Arg Thr His Ser Leu Met Arg
   265 270 275
   Gly Ile Val Gln Gln Gln Asp Asn Leu Leu Arg Ala Ile Gln Ala Gln
   280 285 290
   Gln His Leu Leu Arg Leu Ser Val Trp Gly Ile Arg Gln Leu Arg Ala
   295 300 305
   Arg Leu Gln Ala Leu Glu Thr Leu Met Gln Asn Gln Gln Leu Leu Asn
   310 315 320
   Leu Trp Gly Cys Lys Gly Lys Leu Ile Cys Tyr Thr Ser Val Lys Trp
   325 330 335
   340
   Asn Glu Thr Trp Gly Gly Asn Leu Ser Ile Trp Asp Ser Leu
   Thr Trp
   345 350
   355

## Beschreibung

Die vorliegende Erfindung betrifft ein neues Retrovirus aus der HIV-Gruppe, die gegenwärtig genauer als HIV-Subtyp O bezeichnet wird. Beschrieben wird ein Verfahren zur Züchtung des Retrovirus. Die Erfindung betrifft weiterhin die Gewinnung dieses Retrovirus sowie die Verwendung des Virus für medizinische Zwecke, für die Diagnostik und bei der Herstellung von Impfstoffen.

Retroviren, die zur sogenannten HIV-Gruppe gehören, führen bei damit infizierten Menschen zu Krankheitserscheinungen, die unter dem Sammelbegriff Immunschwäche bzw. AIDS (Acquired Immune Deficiency Syndrome) zusammengefaßt werden.

Epidemiologische Studien belegen, daß das Humane Immunschwäche Virus (HIV) das aetiologische Agens für die überwiegende Mehrheit der AIDS (Acquired Immune Deficiency Syndrome)-Fälle darstellt. Ein 1983 aus einem Patienten isoliertes und charakterisiertes Retrovirus erhielt die Bezeichnung HIV-1 (Barré-Sinoussi, F. et al., Science 220, 868-871 [1983]). Eine Variante von HIV-1 wird in WO 86/02383 beschrieben.

Eine zweite Gruppe von Humanen Immunschwäche Viren wurde 1985 in Westafrika identifiziert (Clavel, F. et al., Science 233, 343-346 [1986]) und als Humanes Immunschwäche Virus Typ 2 (HIV-2) bezeichnet (EP-A-0 239 425). HIV-2 Retroviren unterscheiden sich deutlich von HIV-1, weisen jedoch auch eine Verwandtschaft zu Affen Immunschwäche Viren (SIV-2) auf. Wie HIV-1 führt auch HIV-2 zu einer AIDS-Symptomatik.

Mit ANT70 (J.Vir., 1994, Vol. 68, No. 3, p. 1586-1596) und MVP-5180/91 (J.Vir., 1994, Vol. 68, No.3, p. 1581-1585) wurden kürzlich neuartige HI-Viren beschrieben, die sich nicht in die HIV 1-Subtypen A - F einordnen lassen. Aufgrund der deutlichen strukturellen Unterschiede zu den bekannten HIV 1-Stämmen wurden beide Isolate vorläufig unter dem Subtyp O zusammengefaßt (G. Myers, Los Alamos Data Base), obwohl sie sich voneinander deutlich in der genomischen Nukleotidsequenz unterscheiden.

Es ist ein Charakteristikum der Humanen Immunschwäche Viren, daß sie eine hohe Variabilität aufweisen, die die Vergleichbarkeit der verschiedenen Isolate deutlich kompliziert. Beim Vergleich diverser HIV-1-Isolate treten z.B. in einigen Regionen des Genoms hohe Variabilitäten auf, während andere Genombereiche vergleichsweise konserviert vorliegen (Benn, S. et al. Science 230, 949-951 [1985]). Ein sehr großer Polymorphismus konnte auch für HIV-2 beobachtet werden (Clavel, F. et al., Nature 324, 691-695 [1986]). Die größte genetische Stabilität besitzen Bereiche in den gag und pol Genen, die für strukturell und enzymatisch essentielle Proteine codieren. Im Unterschied dazu weisen einige Regionen im env-Gen sowie die Gene (vif, vpr, tat, rev, nef), die für regulatorische Proteine kodieren, einen hohen Grad an Variabilität auf.' .

Es konnte weiterhin gezeigt werden, daß Antisera gegen HIV-1 auch mit gag und pol Genprodukten von HIV-2 kreuzreagieren, obwohl nur geringe Sequenzhomologien vorlagen. Ebenfalls war die Hybridisierung zwischen diesen beiden Viren wenig signifikant, wenn nicht sehr wenig stringente Konditionen verwandt wurden (Clavel, F. et al., Nature 324, 691-695 [1986]).

Aufgrund der weiten Verbreitung der Retroviren aus der HIV-Gruppe und der Tatsache, daß zwischen dem Zeitpunkt der Infektion und dem Zeitpunkt, zu dem eindeutige Symptome für pathologische Veränderungen erkennbar sind, ein Zeitraum von einigen bis vielen Jahren (2-20) liegt, ist es epidemiologisch von großer Bedeutung, die Infektion mit Retroviren der HIV-Gruppe möglichst frühzeitig und vor allem zuverlässig zu bestimmen. Dies spielt nicht nur eine Rolle bei der Diagnose von Patienten, die Zeichen von Immunschwäche aufweisen, sondern mehr noch bei der Überprüfung von Blutspendern. Es hat sich herausgestellt, daß bei der Verwendung von Retroviren oder Bestandteilen davon des Types HIV-1 oder HIV-2 in Nachweissystemen bei manchen Seren kein oder nur ein schwacher Nachweis von Antikörpern geführt werden kann, obwohl bei den Patienten, von denen die Seren stammen, Zeichen von Immunschwäche auftreten. Mit Hilfe des erfindungsgemäßen Retrovirus aus der HIV-Gruppe ist in bestimmten Fällen ein derartiger Nachweis möglich.

Insbesondere für die Diagnostik stellt die genotypische Verschiedenheit der HIV-Viren ein erhebliches Problem dar. Im Fall der HIV-1-Viren geht man davon aus, daß sich pro Replikationszyklus ein Nucleotid pro Genom verändert.

Aufgrund dieser genetischen Variabilität können die HIV-Viren außerordentlich flexibel auf den *in vivo*-Selektionsdruck antworten und äußerst schnell solche Mutanten erzeugen, die entweder resistent sind gegenüber pharmakologischen Wirkstoffen oder sie können solche Individuen attackieren, die einen gewissen immunologischen Schutz aufgebaut haben (Sharp et al., "Origins and diversity of human immunodeficiency viruses", AIDS 1994, Vol. 8, Suppl. 1; S 27-S 42).

Um die Ausbreitung von Infektionen zu verhindern, insbesondere bei Bluttransfusionen, aber auch bei Organspenden, soll eine Infektion mit einem HIV-Virus möglichst mit 100 %iger Sicherheit bestimmt werden können. Daher ist es erforderlich, auch solche Infektionen diagnostisch nachzuweisen, die von einem Virus verursacht werden, das derzeit nur in bestimmten geographischen Regionen verbreitet ist, das aber - sofern nicht geeignete Vorsichtsmaßnahmen ergriffen werden - sich auch ohne weiteres in Europa oder den Vereinigten Staaten von Amerika verbreiten kann.

Beschrieben wird die Isolierung und Charakterisierung eines neuen Humanen Immunschwäche Virus, im folgenden als MVP-2901/94 bezeichnet, das 1994 aus peripheren Lymphozyten einer 24-jährigen Patientin aus Kamerun isoliert wurde, die Zeichen von Immunschwäche aufwies. Geographisch stammt dieses Retrovirus aus einer Region in Afrika, die zwischen Westafrika mit endemischer HIV-2 und HIV-1 Virusinfektion und Ostafrika mit fast ausschließlicher HIV-1-Verbreitung lokalisiert ist. Gegenstand der vorliegenden Erfindung ist also ein neues Retrovirus der HIV-Subtyp O-Gruppe, welches als MVP-2901/94 bezeichnet wird sowie davon abgeleitete DNS-Sequenzen und Aminosäuresequenzen und diese enthaltende Test-Kits.

MVP-2901/94 läßt sich in den Zellinien MT2 und Jurkat propagieren. Die Isolierung und Vermehrung von Viren wird in dem Buch "Viral Quantitation in HIV Infection, Editor Jean-Marie Andrieu, John Libbey Eurotext, 1991" ausführlich beschrieben.

Zum besseren Verständnis der Unterschiede des erfindungsgemäßen MVP-2901/94 Virus zu den Retroviren HIV-1 und HIV-2 soll zunächst kurz der Aufbau der Immunschwäche verursachenden Retroviren erläutert werden. Im Inneren des Virus befindet sich die RNA in einem kegelförmigen Core, das aus Proteinuntereinheiten zusammengesetzt ist, die die Bezeichnung p 24 (p für Protein) tragen. Dieses innere Core wird von einer Proteinhülle umgeben, die aus dem Protein p 17 aufgebaut ist (äußeres Core) und von einer Glykoproteinhülle umgeben ist, die neben Lipiden, die aus der Wirtszelle stammen, das Transmembranprotein gp 41, und das Hüllprotein 120 (gp 120) enthält. Dieses gp 120 geht dann mit den CD-4-Rezeptoren der Wirtszellen eine Bindung ein.

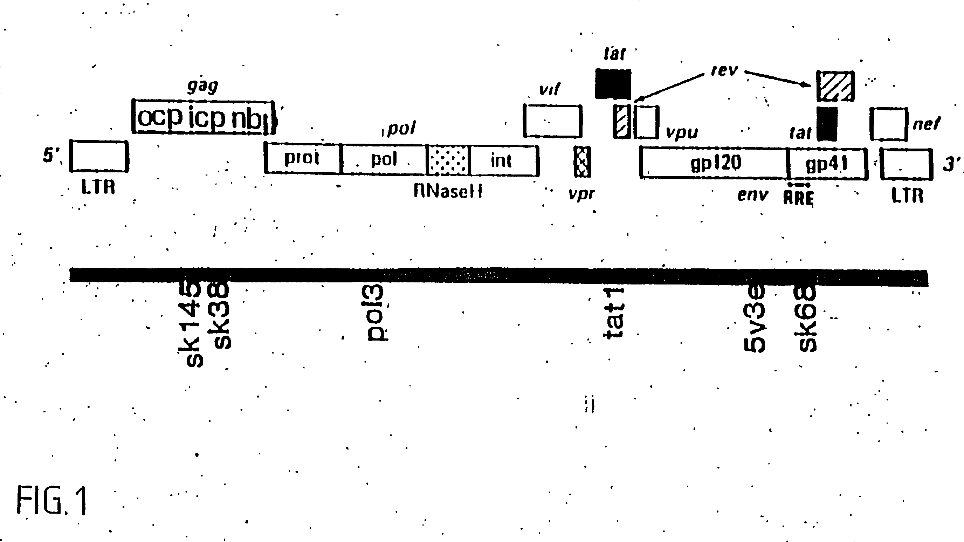

Soweit bekannt, weist die RNA der HIV-Viren - vereinfacht dargestellt - folgende Genbereiche auf: An den beiden Enden sogenannte long terminal repeats (LTR), und die folgenden Genbereiche gag, pol, env und nef. Das Gen gag codiert unter anderem für die Kern(Core)-Proteine, p 24 und p 17, das Gen pol codiert für die Reverse Transkriptase, die Protease, die RNAse H und die Integrase und das Gen env codiert für die Glykoproteine gp 41 und gp 120 der Virushülle. Das Gen nef codiert für ein Protein mit Regulatorfunktion. Eine schematische Anordnung des Genoms von Retroviren des HIV-Typs ist in Figur 1 gezeigt.

Eine sehr vielseitig anwendbare Methode der Gentechnologie ist die sogenannte PCR (polymerase chain reaction) geworden, wobei die zur Durchführung des Verfahrens benötigten Komponenten käuflich erworben werden können. Mit dieser Verfahren ist es möglich, DNA-Sequenzen zu amplifizieren wenn DNA-Bereiche der zu amplifizierenden Sequenz bekannt sind. Es müssen dann kurze komplementäre DNA-Fragmente (Oligonucleotide = Primer) synthetisiert werden, die sich an einen kurzen Bereich der zu amplifizierenden Nukleinsäuresequenz anlagern. Für die Testdurchführung werden HIV-Nukleinsäuren mit den Primern zusammengebracht in einer Reaktionsmischung, die zusätzlich eine Polymerase und Nukleotidtriphosphate enthält. Die Polymerisation (DNA-Synthese) wird für eine bestimmte Zeit durchgeführt und dann werden die Nukleinsäurestränge durch Erwärmen getrennt. Nach Abkühlen läuft dann die Polymerisation erneut an. Wenn es sich also bei dem erfindungsgemäßen Retrovirus um ein HIV-1 oder HIV-2 Virus handelt, dann müßte die Nukleinsäure amplifiziert werden können, indem Primer verwendet werden, die konserviert sind innerhalb der bekannten Sequenzen der Viren HIV-1 und HIV-2. Derartige Primer sind zum Teil vorbeschrieben (Lauré, F. et al., Lancet ii, (1988) 538-541 für pol 3 und pol 4 bzw. Ou C.Y. et al., Science 239 (1988) 295-297 für sk 38/39, sk 68/69).

Diese Primer sind allerdings nicht in der Lage DNA des HIV-Isolates MVP-5180/91 zu amplifizieren(J.Vir., 1994, Vol. 68, No. 3, p. 1581-1585). Der Einsatz dieser Primer führte ebenfalls nicht zu einer Amplifikation von DNA des Isolates MVP-2901/94, was für eine starke Divergenz auch dieses Isolates von der HIV-1-Konsensussequenz sprach. Es mußten also verschiedenste, von bekannten Sequenzen abgeleitete, möglichst stark konservierte Primer neu konstruiert und in möglichst vielen Kombinationen unter Variation der Reaktionsbedingungen eingesetzt werden. Überraschenderweise ergab sich, daß die Kombination der von der Sequenz des Isolates MVP-5180/94 abgeleiteten Primer 212 und 412 unter den in Beispiel 4 aufgeführten Reaktionsbedingungen eine Amplifikation der DNA von MVP-2901/94 ermöglichte und damit einen ersten Einstieg in die Sequenz des Isolates erlaubte.

Nachdem, wie hier geschehen, ein Teilbereich der Sequenz eines HI-Virus entschlüsselt ist, kann nach bekannten Standardmethoden der Molekularbiologie das Gesamtgenom des Virus kloniert und sequenziert werden.
1) Dies kann z.B. durch die Klonierung einer cDNA auf folgendem Weg durchgeführt werden: Das Virus wird aus einer entsprechend großen Kulturmenge (etwa 1 l) präzipitiert und in phosphatgepufferter Kochsalzlösung aufgenommen. Dann erfolgt eine Pelletierung durch ein (20 %iges) Saccharose-Kissen. Das Viruspellet kann in 6 M Guanidiniumchlorid in 20 mM Dithiothreitol und 0,5 % Nonidet P 40 suspendiert werden. CsCl wird bis auf eine Konzentration von 2 molar zugegeben und die das aufgebrochene Virus enthaltende Lösung wird auf ein Cäsiumchlorid-Kissen aufgebracht. Dann wird die virale RNA durch Zentrifugation pelletiert, gelöst, mit Phenol extrahiert und mit Ethanol und Lithiumchlorid präzipitiert. Mit Hilfe eines Oligo(dT)-Primers wird die Synthese des ersten cDNA-Stranges an der viralen RNA oder Teilen davon durchgeführt. Die Synthese unter Zugabe von Reverser Transkriptase kann durchgeführt werden unter Verwendung eines käuflich erhältlichen Kits. Für die Synthese des zweiten Stranges wird der RNA-Strang des RNA/DNA-Hybrids mit RNase H verdaut und der zweite Strang unter Einsatz von E.coli DNA Polymerase I synthetisiert. Mit Hilfe von T4 DNA Polymerase können dann stumpfe Enden erzeugt werden und diese mit geeigneten Linkern für Restriktionsschnittstellen verbunden werden. Nach Restriktionsverdau mit der geeigneten Restriktionsendonuklease wird das cDNA-Fragment aus einem Agarosegel isoliert und mit einem vorher in geeigneter Weise geschnittenen Vektor ligiert. Der Vektor mit dem cDNA-Insert kann dann zur Transformation von kompetenten E.coli-Zellen verwendet werden. Die erhaltenen Kolonien werden dann auf Membranen übertragen, lysiert und denaturiert und schließlich durch Hybridisierung mit Digoxigenin oder Biotin markierter Nukleinsäure aufgefunden. Nach gentechnologischer Herstellung der entsprechenden cDNA ist eine Isolierung der gewünschten DNA-Fragmente, die aus dem Retrovirus stammen, möglich. Durch Einbau dieser Fragmente in geeignete Expressionsvektoren kann dann das gewünschte Protein bzw. Proteinfragment exprimiert werden und für die diagnostischen Tests eingesetzt werden.
2) Alternativ zu der angegebenen Methode kann die Nukleinsäure des Immunschwächevirus mit Hilfe der PCR-Technologie kloniert werden. Dazu müssen jeweils Primer aus dem noch unbekannten Sequenzbereich identifiziert werden, welche in der Lage sind, in Kombination mit von dem bekannten Teil der Sequenz abgeleiteten Primern eine Amplifikation der DNA des Isolates zu ermöglichen.
3) Eine weitere Möglichkeit, ausgehend von dem bekannten Sequenzabschnitt das Virus zu klonieren, ist die Klonierung der proviralen genomischen DNA des Virus. Hierzu wird zunächst genomische DNA einer infizierten Zellinie nach Standardmethoden gereinigt. Die in das Wirtsgenom integrierte provirale DNA kann dann nach Anlage und Screening einer genomischen Bibliothek kloniert werden. Hierzu wird die genomische DNA partiell fragmentiert, die Fraktion der Fragmente einer Länge von etwa 10-25 kB isoliert und in ein Vektorsystem, welches Fragmente dieser Länge aufnehmen kann, wie Cosmide oder Lambda-Phagen, kloniert. Die Mischung der genomischen Fragmente wird mittels des gewählten Vektorsystems in einen E.coli-Stamm transformiert. Durch Hybridisierung mit einem kloniert vorliegenden DNA-Teilstück des gesuchten Virus, welches radioaktiv oder andersartig markiert ist, können dann Vektoren, die das virale Genom enthalten identifiziert und isoliert werden (Plaque- oder Kolonie-Screening). Damit steht dann das virale Genom für eine Sequenzanalyse und eine Expression seiner Proteine zur Verfügung.

Zwischen verschiedenen Virusisolaten kann die Ähnlichkeit ausgedrückt werden durch den Grad der Homologie der Nukleinsäure- oder Proteinsequenzen. Eine 50 %ige Homologie bedeutet beispielsweise, daß 50 von 100 Nukleotid- der Aminosäure-Positionen in den Sequenzen übereinstimmen. Die Homologie von Proteinen wird durch Sequenzanalyse bestimmt. Homologe DNA-Sequenzen lassen sich auch durch die Hybridisierungs-Technik ermitteln.

Gegenstand der vorliegenden Erfindung ist daher ein Immunschwäche-Virus der HIV-Gruppe das bei der European Collection of Animal Cell Cultures (ECACC) unter der Nr. V 95012601 mit der Bezeichnung MVP-2901/94 hinterlegt wurde. Hinterlegungstag ist der 26. Januar 1995.

Für die immunologische Charakterisierung des Virus sind diejenigen Epitope maßgebend, die eine verstärkte Produktion von Antikörpern in infizierten Personen verursachen und die dazu geeignet sind, die Viren in verschiedene Unterklassen und Subtypen einzuteilen. Es handelt sich hierbei also insbesondere nicht um solche Epitope, die auch bei Viren der Gruppen HIV-1 und/oder HIV-2 vorhanden sind, sondern um solche Epitope, die nur bei dem erfindungsgemäßen, hinterlegten Virus auftreten und bei solchen Varianten, die zu der engen Gruppe des Virus MVP-2901/94 gehören. Die morphologischen und immunologischen Eigenschaften des Virus spiegeln sich auch in dem diagnostisch relevanten Bereich des Hüllproteins wieder.

Das erfindungsgemäße Immunschwäche-Virus weist eine RNA-Sequenz auf, die zu der DNA-Sequenz von Tabelle 1 komplementär ist und eine Homologie zu der Sequenz von Tabelle 1 von wenigstens 85 % aufweist.

Gegenstand der vorliegenden Erfindung ist auch eine cDNA, die komplementär ist zu der RNA des bei der European Collection of Animal Cell Cultures (ECACC) unter der Nr. V 95012601 hinterlegten Immunschwäche-Virus MVP-2901/94 oder eines erfindungsgemäßen Virus gemäß Anspruch 1.

Diese cDNA liegt im bevorzugter Ausführungsform in Form von rekombinanter DNA vor.

Die Erfindung umfaßt auch Antigene, die unter Verwendung der erfindungsgemäßen cDNA oder der rekombinanten DNA hergestellt werden oder unter Verwendung der Aminosäurestruktur, die aus seiner cDNA abgeleitet werden kann. Hierbei ist das Antigen ein Protein oder Peptid.

Die erfindungsgemäßen Antigene weisen eine Aminosäuresequenz auf, die der in Tabelle 1 dargestellten Aminosäuresequenz entspricht.

In besonders bevorzugter Ausführungsform weist das erfindungsgemäße Antigen eine Aminosäuresequenz NQQLLNLWGCKGKLICYTSVKWN auf.

Die vorliegende Erfindung umfaßt auch Antigene, die aus einem Immunschwäche-Virus gemäß Anspruch 1 hergestellt werden und beispielsweise in Form von gereinigten Virenpräparationen vorliegen. Bevorzugt wird das erfindungsgemäße Antigen rekombinant hergestellt; es ist aber auch möglich, das Antigen synthetisch herzustellen, beispielsweise durch Festphasensynthese..

Die Erfindung umfaßt auch Testkits zum Nachweis von Antikörpern gegen Immunschwäche .verursachende Viren, die wenigstens ein erfindungsgemäßes Antigen aufweisen.

Bei den Testkits kann es sich um Western Blots, ELISA-Tests oder Fluoreszenz-Antikörper-Nachweistests handeln. Für die Diagnostik von Viren und insbesondere von HIV-Viren hat sich in letzter Zeit herausgestellt, daß solche Verfahren sehr empfindlich und wirkungsvoll sind, bei denen eine Vermehrung (Amplifizierung) der viralen Nukleinsäure, bzw.' eines speziellen Bereichs davon, durchgeführt wird.

Zu den bekannten Nachweisverfahren gehört die Polymerase Kettenreaktion (PCR). Alternativ hierzu kann auch die kompetitive Polymerase Kettenreaktion beim Nachweis von HIV-Infektionen verwendet werden (beispielsweise AIDS (1993), 7, Suppl. 2; S. 65 - S. 71).

Ein anderes Nachweisverfahren, das gerade bei der HIV-Diagnostik in letzter Zeit an Bedeutung gewonnen hat, ist die NASBA (Nucleic Acid Sequence-Based Amplification)-Methode. Dieses Verfahren wird beispielsweise in AIDS 1993, 7 (Suppl. 2): S. 107 - S. 110 beschrieben. Bei diesem Verfahren wird die einzelsträngige RNA oder auch die doppelsträngige DNS mit Hilfe der T7 RNA Polymerase amplifiziert und anschließend nachgewiesen.

Ein weiteres Verfahren zum Nachweis von HIV-Viren ist der Nachweis mit der Signalamplifikation mit Hilfe von verzweigter DNA. Dies wird beispielsweise in AIDS 1993, 7 (Suppl. 2): S. 11 - S. 14 beschrieben. Bei diesem Verfahren wird die virale Nukleinsäure an Proben hybridisiert, die mit einer festen Phase verbunden sind. Weiterhin wird mit der Probe ein Nachweismolekül (verzweigte DNA-Strukturen) hybridisiert und dann enzymatisch nachgewiesen.

Den obigen Verfahren gemeinsam ist, daß bestimmte Nukleinsäurebereiche bei den Nachweisverfahren eingesetzt, werden, die spezifisch sind für das nachzuweisende Virus. Bei diesen Nachweisverfahren werden bestimmte kurze Nukleinsäurefragmente, bei denen es sich insbesondere um DNA-Fragmente handelt, ausgewählt und in den Nachweisverfahren eingesetzt.

Verwendet werden können das erfindungsgemäße Immunschwäche-Virus, die erfindungsgemäße cDNA sowie die Antigene zum Nachweis von Retroviren, die Immunschwäche verursachen.

Insbesondere die erfindungsgemäßen Antigene können zur Herstellung von Impfstoffen verwendet werden.

Gegenstand der Erfindung ist auch Ribonukleinsäure, die für ein erfindungsgemäßes Virus kodiert.

Im Rahmen der vorliegenden Erfindung wurde ein Teil aus dem Hüllprotein sequenziert, der für die Diagnostik von besonderer Relevanz ist. Es handelt sich hierbei um einen Envelop-Bereich, der das Gebiet des sogenannten V3-Loops umfaßt; der im Rahmen der vorliegenden Erfindung sequenzierte Bereich erstreckt sich bis in den sogenannten gp 41-Bereich.

Im Rahmen der vorliegenden Erfindung wurde zunächst ein Teil aus dem Hüllprotein sequenziert und festgestellt, daß diese Sequenz nur eine verhältnismäßig geringe Homologie zu den entsprechenden Sequenzen von Viren vom HIV-Typ aufweist. Insbesondere bezogen auf den gp 41-Bereich wurde durch einen Vergleich mit HIV-Sequenzen, der mit Hilfe von Datenbanken durchgeführt wurde, eine Homologie von höchstens 79,1% auf Nukleotidebene ermittelt.

Das erfindungsgemäße Virus unterscheidet sich durch seine Sequenz von vorbekannten Viren. Gegenstand der vorliegenden Erfindung sind daher solche Viren sowie entsprechende DNS- bzw. Aminosäuresequenzen, die der Sequenz des erfindungsgemäßen Virus weitgehend entsprechen, wobei der Grad der Abweichung durch den Grad der Homologie festgelegt wird. Eine Homologie von beispielsweise mehr als 85 % bedeutet daher, daß solche Sequenzen umfaßt werden, die in wenigstens 85 von 100 Nukleotiden bzw. Aminosäuren dieselben Nukleotide bzw. Aminosäuren aufweisen, während der Rest unterschiedlich sein kann. Bei der Feststellung der Homologie werden die beiden Sequenzen derart gegenübergestellt, daß möglichst viele einander entsprechende Nukleotide bzw. Aminosäuren miteinander zur Deckung kommen.

Anhand der isolierten Sequenz können immundominante Epitope (Peptide) konfektioniert und synthetisiert werden. Da die Nukleinsäuresequenz des Virus bekannt ist, kann der Fachmann hieraus die Aminosäuresequenz ableiten. Ein Teilbereich der Aminosäuresequenz ist in Tabelle 1 angegeben. Offenbart sind daher auch Antigene, d.h. Proteine, Oligo- oder Polypeptide, die mit Hilfe der in Tabelle 1 offenbarten Information hergestellt werden können. Diese Antigene, Proteine, Polypeptide und Oligopeptide weisen Aminosäuresequenzen auf, die in Tabelle 1 angegeben sind. Die Antigene bzw. Peptide können verhältnismäßig kurze Teilsequenzen einer Aminosäuresequenz aufweisen, die in Tabelle 1 wiedergegeben ist. Diese Aminosäuresequenz ist wenigstens 10 Aminosäuren, bevorzugt wenigstens 20 und besonders bevorzugt wenigstens 25 Aminosäuren lang. Hergestellt werden können diese Peptide nicht nur mit Hilfe der rekombinanten Technologie sondern auch durch synthetische Methoden. Ein geeigneter Herstellungsweg ist die Festphasensynthese vom Merrifield-Typ. Eine weitere Beschreibung dieser Technik und anderer im Stand der Technik bekannter Verfahren kann in der Literatur gefunden werden, z.B. M. Bodansky, et al., Peptide Synthesis, John Weeley & Sons, 2nd Edition 1976.

Bei den diagnostischen Tests wird eine Serumprobe der zu untersuchenden Person zusammengebracht mit den Proteinketten von einem oder mehreren Proteinen oder Glykoproteinen (die in eukaryontischen Zellinien exprimiert werden können) oder Teilen davon, die von MVP-2901/94 stammen. Bevorzugte Testverfahren schließen die Immunfluoreszenz oder immunenzymatische Testverfahren (z.B. Elisa, Immunoblot) ein.

Bei den immunenzymatischen Tests (ELISA) kann beispielsweise Antigen, das von MVP-2901/94 oder einer Variante davon stammt, an den Wänden von Mikrotiterplatten gebunden werden. Die dabei verwendete Dosierung hängt von dem Testsystem und der Behandlung der Mikrotiterplatten wesentlich ab. Dann wird Serum bzw. Serumverdünnungen, die von der zu untersuchenden Person stammen, in die Vertiefungen der Mikrotiterplatten gegeben. Nach einer bestimmten Inkubationszeit wird die Platte gewaschen und spezifische Immunkomplexe werden nachgewiesen durch Antikörper, die spezifisch an menschliche Immunglobuline binden und die vorher mit einem Enzym, beispielsweise Meerrettichperoxidase, alkalischer Phosphatase usw. verbunden wurden oder mit enzymmarkiertem Antigen. Diese Enzyme können ein farbloses Substrat in ein stark gefärbtes Produkt umwandeln und an der Stärke der Färbung kann dann das Vorhandensein von spezifischen Anti-HIV-Antikörpern abgelesen werden. Eine weitere Möglichkeit der Verwendung des erfindungsgemäßen Virus in Testsystemen ist die Verwendung in Western Blots.

Auch wenn die Herstellung von Impfstoffen gegen Immunschwächeerkrankungen sich als äußerst schwierig erweist, kann doch auch dieses Virus bzw. Teile davon, d.h. immundominante Epitope und Induktoren der zellulären Immunität, oder gentechnologisch hergestellte Antigene zur Entwicklung und Herstellung von Impfstoffen verwendet werden.

### Beispiel 1 (Inkulturnahme des Virus)

Das erfindungsgemäße Immunschwäche-Virus MVP-2901/94 wurde aus dem Blut einer Patientin mit Zeichen von Immunschwäche isoliert. Hierzu wurden periphere mononukleäre Zellen (peripheral blood lymphocytes, PBL) und periphere Lymphozyten aus dem Blut (PBL) eines nicht HIV-infizierten Spenders mit Phytohämagglutinin stimuliert und in Kultur gehalten. Verwendet wurde hierzu das übliche Medium RPMI 1640 mit 10 % fötalem Kälberserum. Die Kulturbedingungen sind beschrieben in Landay A. et al., J. Inf. Dis., 161 (1990) S. 706-710. Es wurde keine Bildung von Riesenzellen beobachtet. Die Produktion von HI-Viren wurde über die Bestimmung des p 24-Antigens mit Hilfe des käuflich erwerbbaren Tests von Abbott bestimmt. Ein weiterer Test zur Bestimmung des Wachstums der Viren war der Test unter Verwendung von partikelgebundener Reverser Transkriptase (Eberle J., Seibl R., J. Virol. Methods 40, 1992, S. 347-356). Das Wachstum der Viren wurde also anhand der enzymatischen Aktivitäten im Kulturüberstand ein- bis zweimal pro Woche bestimmt, um die Virusproduktion zu überwachen. Wöchentlich einmal wurden neue Spenderlymphozyten zugegeben.

Nachdem eine HI-Virenvermehrung festgestellt werden konnte, wurden frische periphere Lymphozyten aus dem Blut (PBL) nicht HIV-infizierter, gesunder Spender mit dem Überstand der ersten Kultur infiziert. Dieser Schritt wurde wiederholt und dann wurden mit dem Überstand MT2- bzw. Jurkat-Zellen infiziert. Auf diese Weise war eine permanente Produktion des Immunschwäche-Virus möglich.

### Beispiel 2

### DNA Isolierung, Amplifizierung und strukturelle Charakterisierung von Genomabschnitten des HIV Isolates MVP-2901/94 (kodierend für gp 41)

Genomische DNA aus MVP-2901/94-infizierten Blutlymphozyten wurde nach Standardmethoden isoliert (Current Protocols in Molecular Biology, Wiley Interscience, 1994).

Zur Charakterisierung von Genombereichen des Isolates MVP-2901/94 wurden PCR (Polymerase Chain Reaction)-Experimente mit Primerpaaren aus dem Hüllproteinbereich gp 41 durchgeführt. Die PCR (Saiki et al., Science 239: 487-491, 1988) wurde mit folgenden Modifikationen durchgeführt:

Für die Amplifikation HIV-spezifischer DNA Bereiche wurden 5 pl (200 µg/ml) genomische DNA aus MVP-2901/94 infizierten Blutlymphozyten in einem 100 pl Reaktionsansatz (0,25 mM dNTP, je 1 µM Primer, 10 mM Tris HCl, pH 8.3, 50 mM KCl, 1.5 mM MgCl₂, 0,001 % Gelatine, 2,5 units Taq Polymerase (Perkin Eimer)) pipettiert und nach folgendem Temperaturprogramm amplifiziert: 1. Initiale Denaturierung: 3 min. 95°C, 2. Amplifikation: 90 sec. 94°C, 60 sec. 56°C, 90 sec. 72°C (30 Cyclen).

Die für die PCR und die Nukleotidsequenzierung verwendeten Primer wurden auf dem Oligonukleotidsynthesizer 8750 der Firma Biosearch synthetisiert, wobei die Primer folgende Sequenzen aufwiesen:

Nachdem mit in der Literatur beschriebenen Primern (Lauré, F. et al., Lancet ii, (1988) 538-541 für pol 3 und pol 4 bzw. Ou C.Y. et al., Science 239 (1988) 295-297 für sk 38/39, sk 68/69) keine Amplifikation des Isolates möglich war, wurden verschiedenste, von bekannten Sequenzen abgeleitete, möglichst stark konservierte Primer neu konstruiert und in allen denkbaren Kombinationen unter Variation der Reaktionsbedingungen eingesetzt. Überraschenderweise ergab sich, daß die Kombination der von der Sequenz des Isolates MVP-5180/94 abgeleiteten Primer 212 und 412 unter den oben aufgeführten Reaktionsbedingungen eine Amplifikation der DNA von MVP-2901/94 ermöglichte und damit einen ersten Einstieg in die Sequenz des Isolates erlaubt.

Durch Sequenzierung des ersten Amplifikats konnten die MVP-2901/94 spezifischen Primer 425 und 431 entworfen werden. Um den nun bekannten Bereich weiter zu expandieren, wurden neue Primer nach oben genannten Kriterien entworfen und in Kombination mit Primer 425 oder Primer 431 eingesetzt. Eine Expansion in 3'-Richtung gelang dann mit dem von MVP-5180/91 abgleiteten Primer 214 in Kombination mit 425 und eine Expansion in 5'-Richtung mit den Kombinationen 431/438 und 431/447, wobei die Primer 438 und 447 von bei den meisten Subtypen von HIV1 konservierten Bereichen abgeleitet wurden.

Die amplifizierte DNA wurde über ein 3 %. "Nusieve"-Agarosegel (Fa. Biozyme) aufgetrennt, das amplifizierte Fragment ausgeschnitten und mit dem gleichen Volumen an Puffer (1xTBE (0.09 M TrisBorat, 0.002 M EDTA, pH 8.0)) versetzt. Nach Inkubation des DNA-Agarosegemisches für 10 Minuten bei 70°C und nachfolgender Phenolextraktion wurde die DNA aus der wässrigen Phase durch Zugabe von 1/10 Vol 3M NaAc, pH 5,5 und 2 Vol Ethanol bei -20°C 15' gefällt und anschließend in einer Eppendorfzentrifuge pelletiert (13000rpm, 10', 4°C). Die pelletierte DNA wurde getrocknet, in Wasser aufgenommen und nach der photometrischen Bestimmung der DNA-Konzentration bei 260nm im Spektralphotometer (Fa. Beckman) nach der Methode von Sanger (F. Sanger, Proc. Natl. Acad. Sci., 74:5463, 1977) sequenziert. Anstelle der Sequenzierung mit Klenow DNA Polymerase wurde die Sequenzierungsreaktion mit einem Kit der Fa. Applied Biosystems ("Taq Dye Deoxy Terminator Cycle Sequencing", Best. Nr.: 401150) durchgeführt. Als Primer wurden in getrennten Sequenzierungsreaktionen jeweils einer der für die PCR verwendeten Primer (jeweils 1µM) eingesetzt. Die Analyse der Sequenzierungsreaktion erfolgte auf dem DNA-Sequenziergerät 373A (Applied Biosystems) nach den Vorgaben des Geräteherstellers.

Die Nukleotidsequenz des amplifizierten DNA-Bereichs und die davon abgeleitete Aminosäuresequenz sind in der Tabelle 1 dargestellt (Sequ. ID No. 10).

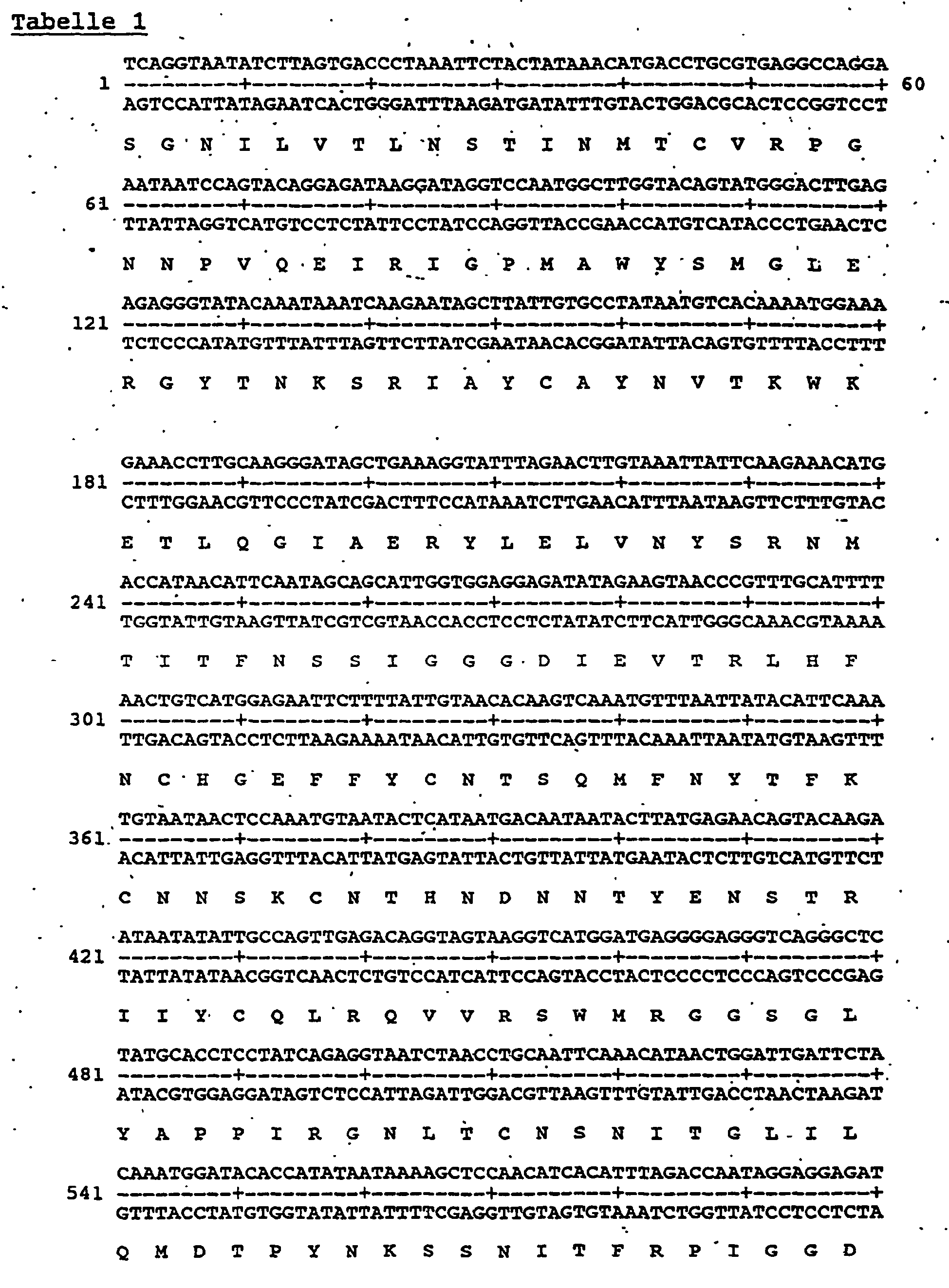

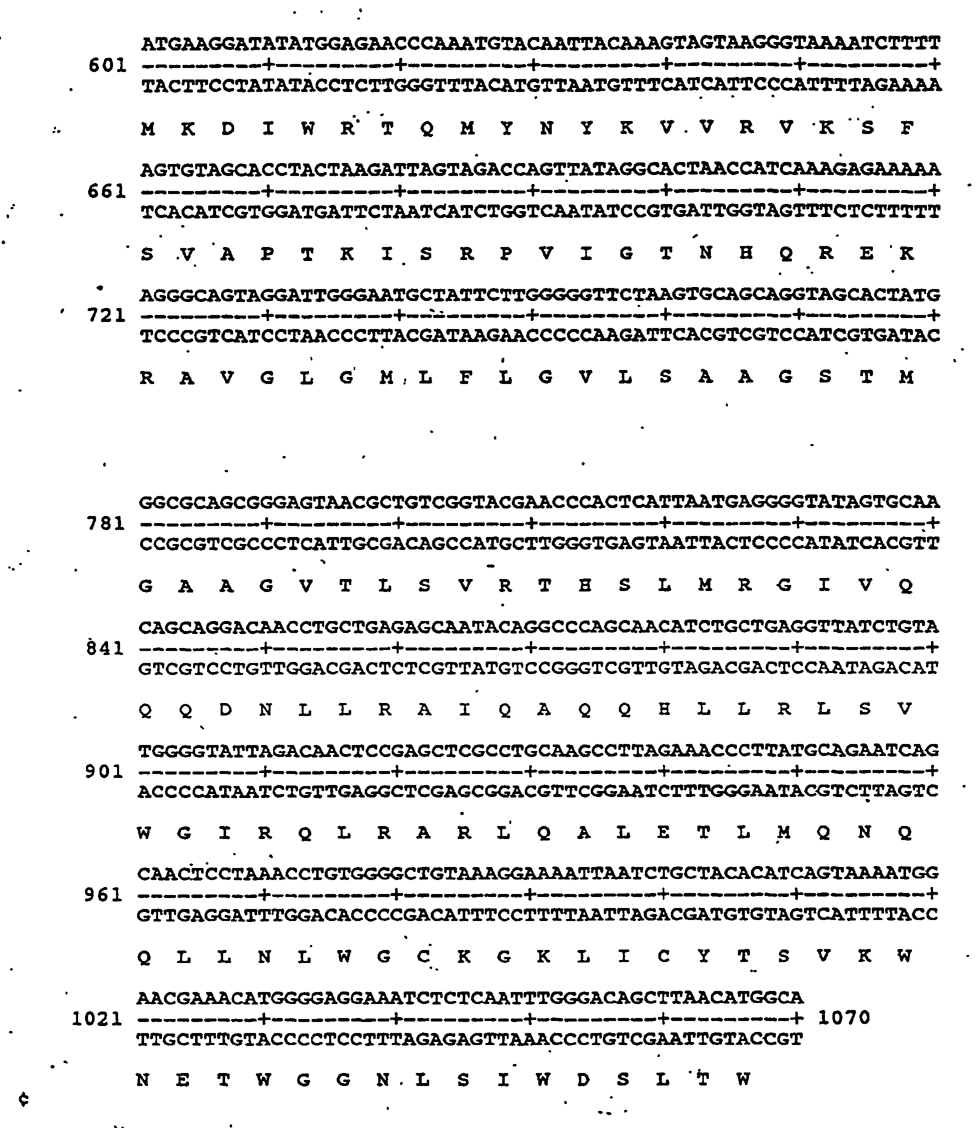

### Beispiel 3

### Abgrenzung des Isolates MVP-2901/94 von anderen HIV-Isolaten

Die gefundene Nukleotidsequenz aus Tabelle 1 wurde auf homologe Sequenzen in der GENEBANK-Datenbank (Release 83, Juni 1994) sowie der EMBL-Datenbank (Release 38, März 1994), die davon abgeleitete Proteinsequenz mit der SWISSPROT Protein-Datenbank (Release 28, Februar 1994) mit Hilfe des GCG-Computerprogramms (Genetic Computer Group, Inc. Wisconsin USA, Version 7.1, März 1992) untersucht. In dieser Datenbank sind die meisten der bis Juli 1994 bekannten Nukleotidsequenzen von Immundefizienzviren humanen Ursprungs und von Isolaten aus Primaten enthalten.

Die Nukleotidsequenz aus Tabelle 1 weist im besten Fall eine 79,6 %ige Homologie zu einem HIV 1-Subtyp O-Isolat auf. Die beste Homologie zu einem anderen HIV1-Subtyp beträgt 59,6%. Zu HIV 2 Isolaten ist die DNA aus Tabelle 1 bestenfalls zu 51,6 % homolog.

Die Aminosäuresequenz aus Tabelle 1 ist im besten Fall mit 72,7% zu dem korrespondierenden Hüllproteinabschnitt eines HIV1-Subtyp-O Vertreters homolog und im besten Fall zu 52,1% zu dem des HIV1-Isolates HIV1-Mal. Zu HIV 2-Hüllproteinen beträgt die Homologie mit der Aminosäuresequenz aus Tabelle 1 im besten Fall 37,0% (Isolat HIV2ROD).

**Tabelle 2 Homologievergleiche von MVP-2901/94 auf Nukleotid- und Proteinebene mit anderen HIV-Isolaten**

| | Beste Homologien zu den HIV1-Subtyp O Vertretern | Beste Homologie zu einem anderen HIV1-Subtyp | Beste Homologie zu einem HIV2-Isolat |
|---|---|---|---|
| Nukleotidebene | 79,1% ANT70 78,0% MVP-5180 | 59,6% HIV1u08450 (SubtypB) | 51,6% HIV2U1GMN |
| Proteinebene | 72,7% ANT70 70,3% MVP-5180 | 52,1% HIV1MAL (Subtyp B) | 37,0% HIV2ROD |

Aufgrund der Homologievergleiche ist das Isolat MVP-2901/94 den beiden vorläufig als HIV1-Subtyp O bezeichneten Isolaten MVP-5180/91 und ANT70 am ähnlichsten. Dennoch besteht zu beiden Isolaten eine relativ hohe Sequenzheterologie von mindestens 20,9% auf Nukleotid- und von mindestens 27,3% auf Proteinebene.

Bei dem Bereich ENV handelt es sich um den diagnostisch relevanten Bereich des Hüllproteins, der in Tabelle 1 sowohl als Nucleotidsequenz wie auch als Aminosäuresequenz angegeben ist.

### Beispiel 4 (Serologische Daten zu MVP-2901/94)

Zur Einschätzung der Bedeutung dieses Virus für die Serodiagnostik, wurde eine Serumprobe des mit 2901 infizierten Patienten in verschiedenen kommerziellen Anti-HIV 1/2-Screening-Tests untersucht.

In Tab. 3 sind die Ergebnisse aus diesen Untersuchungen dargestellt.

**Tabelle 3**

| Probe | Enzygnost Anti-HIV 1/-HIV 2 | Abbott Anti-HIV 1/2, 3. Generation | Ortho/CBC Anti-HIV 1/2 | 2901 - gp41 Peptid |
|---|---|---|---|---|
| 2901 | 0,7 | 0,5 | 0,4 | 4,2 |

Angaben in O.D./Cut off Radio

Aus der Tabelle 3 '.geht hervor, daß keiner der kommerziell erhältlichen Testkits diese Probe erfaßt. Wird dagegen ein neuartiger ELISA eingesetzt, wobei als Festphasenantigen ein Peptid (NQQRLNLWGCKGKLICYTSVKWN) das mit Ausnahme einer Aminosäure (NQQRL statt NQQLL) der 2901-Sequenz entspricht und für die Flüssigreagenzien die Enzygnost Anti HIV1/2 Reagenzien benutzt, so wird die Probe sicher erfaßt. Kommerziell erhältliche Western Blots, wie z. B. der der Fa. Pasteur, erfassen diese Probe MVP2901/94 nicht (ohne Abbildung). Solche Western Blots würden deshalb sehr wahrscheinlich ein falsch negatives Ergebnis liefern, mit Proben, die auf MVP2901/94-Infektion zurückgehen.

Ein besonders bevorzugter Bereich aus der in Tabelle 1 dargestellten Aminosäuresequenz ist der Bereich, der mit der Aminosäuresequenz NQQLL... beginnt (dieser Bereich beginnt etwa beim Nukleotid 1010 gemäß der in Tabelle 1 verwendeten Zählweise.

Das Beispiel 4 belegt auch, daß für die diagnostische Verwertung der Offenbarung der vorliegenden Erfindung geringfügige Änderungen in der Aminosäuresequenz durchgeführt werden können, ohne daß die diagnostische Aussagekraft eines entsprechenden Tests nachteilig beeinflußt wird.

## Patentansprüche

1. Immunschwäche-Virus der HIV-Gruppe, das bei der European Collection of Animal Cell Cultures (ECACC) unter der Nr V 95012601 hinterlegt wurde, mit der Bezeichnung MVP-2901/94 und eine RNA-Sequenz aufweist, die zu der DNA-Sequenz von Tabelle 1 komplementar ist und eine Homologie zu der Sequenz von Tabelle 1 von wenigstens 85 % aufweist

2. cDNA umfassend die Nucleinsäuresequenz der SEQ ID NO.10 oder sein Komplement.

3. Rekombinante DNA, **dadurch gekennzeichnet, dass** sie die cDNA gemäß Anspruch 2 aufweist.

4. Antigen umfassend die Aminosauresequenz von SEQ ID NO:12

5. Testkit zum Nachweis von Antikörpern gegen Immunschwäche verursachende Viren, **dadurch gekennzeichnet, dass** es ein Antigen gemaß Anspruch 4 aufweist.

6. Testkit gemaß Anspruch 5, **dadurch gekennzeichnet, dass** es ein Western Blot ist

7. Testkit gemäß Anspruch 5, **dadurch gekennzeichnet, dass** es ein ELISA-Test oder ein Fluoreszenz-Antikörper-Nachweistest ist

8. Verwendung eines Immunschwache-Virus gemäß Anspruch 1 oder einer cDNA gemaß Anspruch 2 und/oder eines Antigens gemäß Anspruch 4 zum Nachweis von Retroviren, die Immunschwäche verursachen.

9. Verwendung eines Retrovirus nach Anspruch 1, einer cDNA gemaß Anspruch 2 und/oder eines Antigens gemäß Anspruch 4 zur Herstellung von Impfstoffen

10. Ribonukleinsaure, **dadurch gekennzeichnet, dass** sie für das Immunschwäche-Virus nach Anspruch 1 kodiert und eine RNA-Sequenz aufweist, die zu der DNA-Sequenz von Tabelle 1 komplementär ist und eine Homologie zu der Sequenz von Tabelle 1 von wenigstens 85 % aufweist

## Claims

1. An immunodeficiency virus of the HIV group which is deposited with the European Collection of Animal Cell Cultures (ECACC) under No. V 95012601, which has the designation MVP-2901/94 and which exhibits an RNA sequence which is complementary to the DNA sequence depicted in Table 1 and which possesses an homology of at least 85% with the sequence in Table 1.

2. A cDNA which comprises the nucleic acid sequence set forth in SEQ ID NO:10 or its complement.

3. A recombinant DNA which comprises cDNA as claimed in claim 2.

4. An antigen which comprises the amino acid sequence set forth in SEQ ID NO:12.

5. A test kit for detecting antibodies against viruses causing immune deficiency, which contains an antigen as claimed in claim 4.

6. Test kit as claimed in claim 5, which is a Western Blot.

7. Test kit as claimed in claim 5, which is an ELISA test or a fluorescence antibody detection test.

8. The use of an immunodeficiency virus as claimed in claim 1, or of a cDNA as claimed in claim 2, and/or of an antigen as claimed in claim 4, for detecting retroviruses which cause immune deficiency.

9. The use of a retrovirus as claimed in claim 1, of a cDNA as claimed in claim 2, and/or of an antigen as claimed in claim 4, for preparing vaccines.

10. A ribonucleic acid which encodes an immunodeficiency virus as claimed in claim 1 and which comprises an RNA sequence which is complementary to the DNA sequence depicted in Table 1 and which possesses an homology of at least 85% with the sequence in Table 1.

## Revendications

1. Virus immunodéficient du groupe du VIH qui a été déposé auprès de la European Collection of Animal Cell Cultures (ECACC) sous le n° V 95012601, avec la désignation MVP-2901/94 et qui présente une séquence d'ARN complémentaire à la séquence d'ADN du tableau I et une homologie d'au moins 85 % par rapport à la séquence du tableau I.

2. ADNc comprenant la séquence d'acide nucléique de la SEQ ID n° 10 ou son complément.

3. ADN recombinant, **caractérisé en ce qu'**il présente l'ADNc selon la revendication 2.

4. Antigène comprenant la séquence d'acides aminés de la SEQ ID n° 12.

5. Trousse d'essai pour la détection d'anticorps contre des virus provoquant une immunodéficience, **caractérisée en ce qu'**elle met en évidence un antigène selon la revendication 4.

6. Trousse d'essai selon la revendication 5, **caractérisée en ce qu'**elle constitue un transfert western.

7. Trousse d'essai selon la revendication 5, **caractérisée en ce qu'**elle constitue un test ELISA ou un test de détection d'anticorps par fluorescence.

8. Utilisation d'un virus immunodéficient selon la revendication 1 ou d'un ADNc selon la revendication 2 et/ou d'un antigène selon la revendication 4 pour détecter des rétrovirus qui provoquent une immunodéficience.

9. Utilisation d'un rétrovirus selon la revendication 1, d'un ADNc selon la revendication 2 et/ou d'un antigène selon la revendication 4 pour la fabrication de vaccins.

10. Acide ribonucléique, **caractérisé en ce qu'**il code pour le virus immunodéficient selon la revendication 1 et **en ce qu'**il présente une séquence d'ARN complémentaire à la séquence d'ADN du tableau I et présentant une homologie d'au moins 85 % par rapport à la séquence du tableau I.
